(19) **Europäisches Patentamt**
**European Patent Office**
**Office européen des brevets**

(11)   **EP 2 157 167 B1**

(12)   **EUROPEAN PATENT SPECIFICATION**

(45) Date of publication and mention
of the grant of the patent:
**28.09.2011  Bulletin 2011/39**

(51) Int Cl.:
*C12N 1/12* [(2006.01)]   *A23K 1/16* [(2006.01)]
*A23L 1/275* [(2006.01)]   *C12P 23/00* [(2006.01)]
*A61K 36/05* [(2006.01)]

(21) Application number: **08014415.7**

(22) Date of filing: **13.08.2008**

(54) **Process for obtaining Lutein from green algae**

Verfahren zur Erzeugung von Lutein aus grünen Algen

Procédé pour obtenir de la lutéine à partir d'algue verte

(84) Designated Contracting States:
**AT BE BG CH CY CZ DE DK EE ES FI FR GB GR
HR HU IE IS IT LI LT LU LV MC MT NL NO PL PT
RO SE SI SK TR**

(43) Date of publication of application:
**24.02.2010  Bulletin 2010/08**

(73) Proprietors:
• **Cognis IP Management GmbH
40589 Düsseldorf (DE)**
• **UNIVERSIDAD DE SEVILLA
ES-41012 Sevilla (ES)**

(72) Inventors:
• **Vargas, Angeles M.
41700 Dos Hermanas, Sevilla (ES)**
• **Martin, Lucia
41016 Sevilla (ES)**
• **Cordero, Baldomero F.
41930 Bormujos, Sevilla (ES)**
• **Obraztsova, Irina
41010 Sevilla (ES)**
• **Rodriguez Martinez, Herminia, Prof. Dr.
41018 Sevilla (ES)**
• **Verseck, Stefan, Dr.
40721 Hilden (DE)**
• **Gerfertz-Martinez, Claudia, Dr.
47441 Moers (DE)**
• **Gutsche, Bernhard, Dr.
40724 Hilden (DE)**
• **Weiss, Albrecht, Dr.
40764 Langenfeld (DE)**

(56) References cited:
**EP-A- 1 808 483     US-A- 3 280 502**

• **MATSUKAWA R ET AL: "Antioxidants from carbon dioxide fixing Chlorella sorokiniana" JOURNAL OF APPLIED PHYCOLOGY, vol. 12, no. 3-5, October 2000 (2000-10), pages 263-267, XP002376926 ISSN: 0921-8971**
• **DATABASE MEDLINE [Online] US NATIONAL LIBRARY OF MEDICINE (NLM), BETHESDA, MD, US; July 1984 (1984-07), LALUCAT J ET AL: "Utilization of light for the assimilation of organic matter in Chlorella sp. VJ79." XP002515096 Database accession no. NLM18553430 & BIOTECHNOLOGY AND BIOENGINEERING JUL 1984, vol. 26, no. 7, July 1984 (1984-07), pages 677-681, ISSN: 0006-3592**
• **DEL CAMPO J A ET AL: "Carotenoid content of chlorophycean microalgae: factors determining lutein accumulation in Muriellopsis sp. (Chlorophyta)." JOURNAL OF BIOTECHNOLOGY. 7 JAN 2000, vol. 76, no. 1, 7 January 2000 (2000-01-07), pages 51-59, XP002376925 ISSN: 0168-1656**

## Description

### Field of the invention

The present invention is related to the area of biotechnology and refers to a new process for obtaining lutein from certain mutants of green algae and the use of said mutants for obtaining lutein.

### Background of the invention

Carotenoids are synthesized by all photosynthetic organisms as well as by some non-photosynthetic bacteria and fungi. There are two main classes of naturally occurring carotenoids: carotenes, which are hydrocarbons that are either linear or cyclized in one or both ends of the molecule and xanthophylls, which are oxygenated derivatives of carotenes. In microalgae a distinction can be made between primary and secondary carotenoids. Primary carotenoids, as lutein, function as accessory pigments in the photosystems, as structural components of light harvesting complexes in chloroplasts, as well as photoprotective agents and therefore are essential for cellular survival. Secondary carotenoids, as astaxanthin, accumulate in large quantities in lipid bodies outside the chloroplasts, after subjecting cells to stress conditions. The role of secondary carotenoids in algal cells is not fully understood. They could function as photoprotective filters and as antioxidants preventing accumulation of oxygen radicals.

Lutein is used as food dye and especially as feed additive in aquaculture and poultry farming; it is also used for the coloration of drugs and cosmetics. During last years, additional applications for lutein, especially in the field of human health, have been found. Lutein is used as a neutraceutical against macular degeneration. Lutein and zeaxanthin are known to play a critical function in maintaining a normal visual function. In addition to the development of cataracts, also the progression of early atherosclerosis seems to be hampered by lutein. In the canine and cats, it has been proofed that lutein enhances both cell mediated and tumoral immune response (Kim et al. 2000). Lutein may also serve to protect skin from UV-induced damage. Global lutein market has been increasing markedly in the last years and is expected to reach US $ 187 million in 2009. Currently the commercial source of lutein is Marigold (*Tagetes erecta* and *Tagetes patula*). However, the lutein content of Marigold flowers is low (0.3 mg g $^1$ DW), and therefore there is an increasing interest in microalgae as an alternative source of this carotenoid. For example, the micro-algae *Muriellopsis* sp., *Chlorella zofingiensis*, *Scenedesmus almeriensis* and *Chlorella protothecoides* have already been proposed as potential sources of lutein. Nevertheless, the described lutein productivities are not high enough to be economically feasible on an industrial scale.

EP-A-1 808 483 discloses a process for obtaining lutein from algae, using Chlorella sorokiniana (SAG 211-32).

Therefore the problem underlying the present invention has been to serve the need to improve lutein accumulation and productivity, by selecting an adequate strain, optimizing culture conditions and obtaining super-producing lutein mutants.

### Detailed description of the invention

The present invention refers to a process for obtaining Lutein from algae which is characterised in that

(a) green algae selected from a group of mutant strains of *Chlorella sorokiniana* (SAG 211-32) identified by the CCAP deposition references

(i) CCAP 211-93
(ii) CCAP 211-94
(iii) CCAP 211-95
(iv) CCAP 211-96

are cultivated to produce lutein until a desired content of the antioxidant is reached,
(b) the algae are harvested and used as a product and/or
(c) the content of lutein is separated off from the remaining biomass.

In order to avoid any ambiguity it is set out that the mutant strains (i) to (iv) have been deposited prior to the filing day of the present application with the Culture Collection of Algae and Protozoa (CCAP) under the given accession numbers, all under the terms of the Budapest Treaty. The algae strains are thus fully disclosed and open for the public.

Surprisingly it has been observed that the mutants of *Chlorella sorokiniana* exhibit an increased Lutein productivity compared to the wild form. In particular, the mutants show a higher content in lutein, keeping the same growth rate as in the wild strain.

Mutagenesis

In case of artificial mutagenesis there are several options known in the art. Mutations can be induced on the basis of chemical or physical mutagens as well as on the basis of biological active substances. Among the chemical, physical and biological mutagens several useful compounds are known in the art from which the following are particular useful.

For instance such mutagens or noxa are of particular interest which are selected from the group consisting of intercalating agents, alkylating agents, deaminating agents, base analogs, electromagnetic radiation comprising radioactive radiation, and x-rays, ionizing radiation, ultraviolet-light or elevated temperature, biological active substances comprising transposons including known technologies connected therewith comprising gene recombinant technologies, transposon mutagenesis and the like. However, mutations based on a spontaneous mutation are also included in this context. In particular, among the intercalating agents the acridine derivatives or the phenanthridine derivatives such as ethidium bromide also known as 2,7-diamino-10-ethyl-6-phenylphenanthridium bromide or 3,8-diamino-5-ethyl-6-phenylphen-antridinium bromide are well known. As to the alkylating agents compounds such as nitrosoguanidine derivatives or ethyl methanesulfonate; ethyl ethanesulfonate, nitrous acid, or $HNO_2$ are also useful. However, regarding the nitrosoguanidine derivatives the compound N-methyl-N'-nitro-nitrosoguanidine is of particular interest in order to carry out the present invention. Concerning the base analogs the compound 5-bromo-uracil, which is also known as deoxynucleosid 5-bromodeoxyuridine, or 2-aminopurine can be used.

Among the transposons there are a lot of methods published in the literature that describes transposon mutagenesis. For example, retrotransposons or DNS transposons, for example Mu phage transposon, or transposon-tagging, are well known and are available for the induction of mutations. In addition, site-directed mutagenesis can also be achieved by using known technologies such as linker-insertion mutagenesis, generation of deletion mutants or oligonucleotide-directed mutagenesis. In general, all known biological methods based on gene recombination are included within the context of the above methods. Because the skilled person is aware of the great variety of the adequate literature there is no need to cite specific ones.

The micro-organisms to be mutagenized can be exposed to the mutagen or the mutation inducing agents or noxa according to any known methods. However, according to the present invention, it is preferred that artificial mutagenesis is performed with at least one chemical mutagen, in particular with an alkylating compound, more particular with a nitroso-compound, especially with a nitrosoguanidine derivative. Among the nitrosoguanidine derivatives the compound N-methyl-N'-nitro-nitrosoguanidine is preferred. In general mutagenesis can be carried out by exposing a suitable quantity of cells of the photoautotrophic algae to be mutagenized to a mutagen or mutagen-inducing agent within an appropriate time and conditions.

In particular, mutagenesis can be performed by suspending cells to be mutagenized in a sterilized buffer or in sterilized water in an amount between $10^7$ to $10^9$ cells, for example exhibiting a density of about $10^8$ cells, or by spreading them on an agar plate and exposing them to the mutagen. In case of a liquid medium in which the cells are suspended it has been found that an alkylating agent, in particular a nitrosoguanidine derivative, preferably N-methyl-N'-nitro-nitrosoguanidine (NG) is effective for producing mutants, which are of particular value for the purpose of the present invention. Mutagenesis can be performed in a liquid medium as mentioned above in suitable flasks of a volume between 5000 ml and 1 ml or between 1000 ml and 2 ml between 0°C and 40°C, in particular between 5°C and 30°C, preferably at a temperature between 20° and 25°C, that means at room temperature. In case of a physical mutagen such as x-ray or UV-light culturing on an agar plate is preferred.

The time of exposition of the cells to the mutagen depends on the nature of the mutagen to be used. In particular, if a chemical mutagen is used, such as a nitrosoguanidine derivatives, for example N-methyl-N'-nitro-nitrosoguanidine (NG), the time of contacting the cells with the mutagen is between 1 minute and 24 hours, preferably between 30 minutes and 6 hours, more preferably between 1.0 and 3.0 hours, for example one hour. The concentration of the chemical mutagen may easily be chosen on the basis of the viability of the cells to the mutagen. In case of a chemical mutagen such as an alkylating agent as mentioned above, for example N-methyl-N'-nitro-nitrosoguanidine, it can be advisable to conduct a series of simple tests in order to determine the viability rate of the cells to be mutagenized.

It has been found particular useful obtaining the lutein super-producing mutants from classical mutagenesis using N-methyl-N'-nitro-nitrosoguanidine and a consecutive selection strategy based on resistance to herbicides (norflurazon and nicotine), as well as on the basis of a high growth rate. More particular, the mutants have been obtained from *Chlorella sorokiniana* strain SAG 211/32, using N-methyl-N'-nitro-nitrosoguanidine (NG) and selected on the basis of the resistance of mutated cells to the herbicides norflurazon or nicotine.

Based on the results four mutants resistant to norflurazon or nicotine were selected with respect to growth and lutein content. The best mutant MR-16, resistant to nicotine exhibits an increase in the volumetric lutein yield of 106 % and in dry weight lutein content of 42 % at the end of deceleration phase as compared to the wild type strain. These four mutants showed similar or even better growth than the wild type strain, which indicates that can attain higher lutein productivities.

Cultivation conditions

Many culture conditions and culture media are known for small-scale stock cultures and large-scale cultures of algae cells. However, for the purposes of the present invention, it was found that the optimal culture conditions and media are actually the relatively simple culture conditions and media described herein below, these therefore being the preferred ones in accordance with the invention. For example, temperature is always a critical factor for the growth of algae. It has been found that very favourable conditions are achieved between 20 and 40 °C with an optimum at about 30 °C.

In a further preferred embodiment of the invention, the algae are grown mixotrophically (with additional nutrients to enhance cell growth), more particularly, Amon medium has been found to support the growth in an optimal manner, preferably if combined with nitrates in amounts of 10 to 60 mM, preferably about 40 mM. The growth rate can also be increased by the addition of salts of acetic acid, in particular sodium acetate, in amounts of 20 to 60 mM, preferably about 40 mM.

Finally, irradiation is also a critical parameter. As far as screening and pilot plant tests are concerned, mercury halide lamps are used and cultivation is carried out under a light irradiation of 200 to 1,500, and preferably of about 700 $\mu Em^{-2}s^{-1}$. For production, of course, natural light conditions are preferred.

In a further preferred embodiment of the present invention, the cells are cultivated in a photo-bioreactor, preferably a tubular or a panel photo-bioreactor, having the advantage of a very large surface area with respect to its volume for optimal large-scale production of algae cells in their growth phase. Usually, such bioreactor modules have a volume in the range from 100 to 35,000 litres; depending on the scale of production that is desired, and which as integral components contain commercially available tubes made of PVC, acrylic (plexiglass), polycarbonate or glass, having an inner diameter of about 3 to 5 cm. In operation, the culture cells are circulated through the device in tap water to which air with $CO_2$ is added, using a pump. The green cultures from the liquid stock cultures (or inoccula) are inoculated into said photo-bioreactor, in which the algae are sparged with a $CO_2$ containing gas like a mixture of $CO_2$ in air or $CO_2$ as the main gas component. Sparging and pumping also served to prevent clumping of the cells. For production scale in a tubular photo-bioreactor, 90 to 100% pure $CO_2$ might be applied, also inexpensive $CO_2$ from industrial plants (e.g. from a quick lime process) are preferred.

The initial cell concentration in the photo-bioreactor during the process of cultivation is preferably adjusted from about 0.1 to 0.3 * 10^6 cells/ml at cell concentrations (dry mass) between 0,1 - 0.4 g dry biomass /1, by dilution with fresh modified cultivation medium. The light intensity is usually kept in the range of between 200 and 1,500 $\mu E*m^{-2}s^{-1}$ as provided by mercury halide lamps or as natural light. It has been found advantageous to maintain the temperature in the photo-bioreactor in the range between 25 and 28 °C. By using a glass house as an indoor cultivation room it is easy to keep temperatures below 32 °C in central and northern Europe in summer time. In addition, the tubular photo-bioreactor and the indoor placement have the advantage of clean, long term and controlled operation, and if the photo-bioreactor is made of glass tubes instead of plastic it does not require extensive maintenance or revamping in order to operate for a long time. Further, the culture conditions are extremely inexpensive as the cells are grown in a cheap mineral medium of tap water with the addition of carbon dioxide as, essentially, the major nutrient source.

It should be set out that the yields of Lutein can be further improved in case cultivation of the strains is conducted under stress factors, like for example increased radiation, reduction in the amount of nutrients or addition of chemicals like e.g. peroxides.

Harvesting and recovery of lutein

In order to obtain lutein or a lutein-enriched product from the cultivated algae cells, many procedures have been described, for example, the procedures in WO 89/006910**.** While these procedures may be employed in accordance with the present invention, the preferred procedure is that of centrifugation, or sedimentation or filtration under vacuum to concentrate the cells, and drying of the concentrated cells. The dried cell mass is then preferably stored at low temperatures (e.g., - 20°C or even lower) under oxygen-free conditions, e.g., by vacuum packing or, preferably, by introduction into plastic bags together with nitrogen ($N_2$) to remove the oxygen.

In another preferred embodiment of the invention, the process comprises the following - additional - steps:

(i) Harvesting the cells cultivated in step (b) by collecting said cells to form a concentrated suspension,
(ii) optionally adding antioxidants and emulsifiers to said suspension, and optionally
(iii) disrupting the collected cells and drying them to obtain a lutein or a lutein-enriched product.

Generally, the collection and concentration of said cells is carried out by centrifugation, or sedimentation or filtration under vacuum, and the drying of said cells is done by lyophilisation, combined drying and grinding (air-vortex-mill), or spray drying.

More particularly, while each cultivation cycle optimally lasts about four to six days, the following harvesting procedure is performed based on the fact that algae cells readily sediment once collected from the photo-bioreactor. Thus, the cell biomass from the photo-bioreactor is collected into a standard large volume funnel, e.g. an Imhoff funnel, and left to stand for a few hours (about 3-5 hours) to facilitate sedimentation of the cells. It was found that approximately 30 % b.w. of the total volume of biomass from the bioreactor represented the cell sediment while the remaining approx. 70 % b.w. of the total collected volume represented the tap water used in the cultivation. This tap water can thus be easily collected and used for a new bioreactor inoculation and cultivation procedure (i.e., the originally used tap water is almost completely recyclable). The above precipitated and concentrated cell culture is then collected from the funnel and subjected to centrifugation or vacuum filtration to further concentrate the cells. Routinely, a biomass yield of about 40 % b.w. solids is obtained following the centrifugation step, or about 30 % b.w. solids following vacuum filtration. Here, too, the approximately 60 % b.w. of the total volume subjected to centrifugation, or approximately 70 % b.w. of the total volume subjected to vacuum filtration, being the supernatant volume, could also be collected and used for another round of the cultivation procedure, this supernatant being primarily the original tap water used in the procedure.

The concentrated cell slurry obtained from the above centrifugation step is then homogenized and stabilised by adding anti-oxidants and then dried, preferably by lyophilisation, although spray drying also proved to be effective. Such antioxidants are selected from the group consisting of ethoxyquin, butylated hydroxyanisole, butylated hydroxytoluene (BHT), tocopherols, di-tert-butyl-paracresol and propyl gallate. The preferred antioxidant, however, is a natural tocopherol product containing 30 % b.w. of alpha tocopherol to have at least a real natural product. Usually, the amount of antioxidant added in the grinding procedure will range from about 0.05 to 5 % (w/w) of the amount of dry powder. The powders are packed into a plastic bag pre-filled with nitrogen gas to remove oxygen (which causes pigment oxidation, i.e. degradation of the active) and are then stored at -20 ˚C prior, e.g., to processing to prepare the food additive.

The final stage of producing a lutein or a lutein-enriched product in the form of small particles easily digested by humans or animals may also be carried out in a number of ways as previously described in the art. Thus, lutein and other algae components are processed to assure a high bioavailability. The preferred procedure involves the use of a standard ball mill in which the biomass slurry is disintegrated as a suspension in water in the presence of any suitable anti-oxidant to prevent oxidation of the lutein. After drying, this yields a powder-like product of small particle size.

The powder thus obtained may then be utilized directly or in a mixture with other ingredients as an additive to fish meal for the sake of coloration or in food applications like dietary supplements. In another process, the lutein can be concentrated by an extraction process including extraction with supercritical solvents to use them for formulation of food supplement or pharmaceutical products.

**Industrial application**

According to the teaching of the present invention, certain mutant algae strains have been found to exhibit a surprisingly high productivity for the production of lutein, especially if cultivated under optimised conditions. Therefore, another object of the present invention is directed to the use of green algae selected from a group of mutant strains of *Chlorella sorokiniana* (SAG 211-32) identified by the CCAP deposition references

    (i) CCAP 211-93,
    (ii) CCAP 211-94,
    (iii) CCAP 211-95, and
    (iv) CCAP 211-96

for the production of lutein or lutein-enriched products.

**Examples**

1.

Organism and culture conditions

*Chlorella sorokiniana* SAG 211-32 was obtained from the Culture Collection of Göttingen University (Germany). Cells were grown photoautotrophically by bubbling through the cell suspension air supplemented with 1 % (v/v) $CO_2$ as the only source of carbon, except in the experiments to study the mixotrophic growth, in which the medium was supplemented with 20-60 mM sodium acetate and/or 100 mM glucose. The culture medium of Arnon et al. (1974) modified to contain 4 mM $K_2HPO_4$ and 20 mM $NaNO_3$, except where indicated, was used. The cells were grown in batch culture at 28˚ C (except in the experiments addressed to study the effect of temperature), in Roux flasks of 1 L capacity, laterally and continuously illuminated with mercury halide lamps at 460 $\mu$mol photons m$^{-2}$ s$^{-1}$ (except where indicated) measured at the surfaces of the flasks using a LI-COR quantum sensor (model L1-1905B, Lic-Cor, Inc. Lincoln, NE, USA) connected to a quantum photometer.

2.

Mutagenesis and selection of lutein-super-producing mutants

Cells in the logarithmic growth phase ($10^6$ cells mL$^{-1}$) were harvested by centrifugation (2,700 x g, 10 min), washed with steril water and treated with 0.1 mg mL$^{-1}$ N-methyl-N-nitro-N-nitrosoguanidine (NNG) (survival 5-10 %) for 1 h. The treated cells were washed with sterile water, resuspended in Amon modified medium and incubated under dim light during 24 h. Cells were spread on solid modified Arnon medium containing, either 4 $\mu$M norflurazon or 400 $\mu$M nicotine and incubated at 25 ˚C and 60 $\mu$mol photons m$^{-2}$ s$^{-1}$ for 3-4 weeks. The herbicide-resistant colonies were subcultivated several times, discarding those showing slow growth. Pre-selected mutants were grown in 100 ml-capacity erlenmeyers under photoautotrophic conditions without acetate and glucose, shaked at 100 rpm and illuminated from the top at 50 $\mu$mol photons m$^{-2}$ s$^{-1}$ to analyze carotenoid content and growth.

3.

Analysis

For carotenoid analysis, pigments were extracted with methanol at 70 ˚C, centrifuged, the supernatant evaporated under $N_2$ and the pellet re-suspended in methanol (Del Campo et al. 2001), centrifuged and analysed by HPLC using a Waters Spherisorb S5 ODS1 (4.6 x 250 mm) cartridge column. The chromatographic method described by Baroli et al. (2003) was used. Pigments were eluted at a flow rate of 1.2 ml/min with a linear gradient from 100 % solvent A (acetonitrile:methanol:0.1 mM Tris-HCl, pH 8.0 [84:2:14]) to 100 % solvent B (methanol:ethyl acetate [68:32]) for 15 min, followed by 3 min of solvent B. The carotenoids were detected at 440 nm using a Waters 2996 photodiode-array detector. For dry weight (DW) determination 5 ml aliquots of the cell culture were filtered through Whatman GF/C paper, washed three times, and the filters containing the algae were dried at 80 ˚C for 24 h. Independent triplicate analyses were carried out for each sample, the results representing the mean values.

4.

Determination of maximum specific growth rate and maximum lutein productivity

Maximum specific growth rate ($\mu_{max}$) was calculated from the measured dry weight, during the logarithmic phase of growth, using the equation:

$$\mu_{max} = (\ln x_2 - \ln x_1) / (t_2 - t_1)$$

where $x_2$ and $x_1$ represent dry weight values in terms of g L$^{-1}$ at times $t_2$ and $t_1$, respectively. Maximum lutein productivity was calculated by multiplying $\mu_{max}$ and the maximal lutein content in the exponential phase (mg L$^{-1}$).

Only the well growing resistant colonies were analysed on lutein content. For the evaluation of lutein production by the obtained mutants we grew the mutants and the corresponding wild strain in liquid cultures with agitation (90 rpm), starting at the same cell density ($5 \times 10^4$ cells/ml). The cells were illuminated at an irradiance of 60 $\mu$E m$^{-2}$ s$^{-1}$. Lutein volumetric yield, lutein content per dry weight of the cultures of best mutants, as well as growth in terms of dry weight of the wild strains and the best mutants are shown in Table 1:

### Table 1

| Comparative analysis of growth and lutein volumetric and dry weight contents of mutants and wild type (WT) strains of *Chlorella sorokiniana* SAG 211-32 in liquid cultures | | | | | | |
|---|---|---|---|---|---|---|
| **Strain** | **Middle deceleration phase** | | | **End deceleration phase** | | |
| | Dry weight (g L$^{-1}$) | Lutein (mg L$^{-1}$) | Lutein (mg g$^{-1}$ DW) | Dry weight (g L$^{-1}$) | Lutein (mg L$^{-1}$) | Lutein (mg g$^{-1}$ DW) |
| **Example 1** | | | | | | |
| WT | 0.96±0.02 | 1.76±0.12 | 1.85±0.24 | 1.09±0.08 | 4.00±0.58 | 3.65±0.30 |
| B7 **(NIC)** | 0.82±0.09 | 2.17±0.21 | 2.68±0.33 | 0.95±0.05 | 4.09±0.25 | 4.30±0.06 |
| B8 **(NIC)** | 0.88±0.18 | 2.21±0.27 | 2.55±0.25 | 1.12±0.16 | 4.68±0.76 | 4.19±0.07 |
| **Example 2** | | | | | | |
| WT | 0.53±0.02 | 2.10±0.10 | 3.96±0.21 | 1.25±0.09 | 4.63±0.15 | 3.70±0.23 |
| K-35 **(NF)** | 0.52±0.10 | 2.45±0.06 | 4.79±0.76 | 1.21±0.03 | 5.82±0.25 | 4.82±0.37 |
| **Example 3** | | | | | | |
| WT | 0.69±0.02 | 2.61±0.21 | 3.76±0.24 | 0.88±0.07 | 4.27±0.45 | 4.84±0.23 |
| K-54 **(NF)** | 0.76±0.01 | 3.53±0.11 | 4.64±0.23 | 1.01±0.05 | 6.00±0.57 | 5.94±0.15 |
| **Example 4** | | | | | | |
| WT | 0.51±0.10 | 1.75±0.06 | 3.57±0.57 | 0.94±0.06 | 3.57±0.58 | 3.79±0.33 |
| MR-3 **(NF)** | 0.58±0.04 | 1.81±0.22 | 3.18±0.80 | 1.23±0.08 | 5.40±0.01 | 4.43±0.28 |
| MR-14 **(NIC)** | 0.52±0.07 | 1.63±0.21 | 3.14±0.02 | 1.19±0.01 | 5.80±0.56 | 4.89±0.50 |
| MR-15 **(NIC)** | 0.64±0.00 | 2.03±0.16 | 3.17±0.25 | 1.08±0.09 | 5.16±0.46 | 4.77±0.03 |
| MR-16 **(NIC)** | 0.73±0.06 | 2.27±0.11 | 3.16±0.54 | 1.33±0.03 | 7.18±1.02 | 5.38±0.91 |
| **Example 5** | | | | | | |
| WT | 0.48±0.06 | 1.54±0.11 | 3.21±0.22 | 0.88±0.03 | 3.76±0.57 | 4.24±0.48 |
| DMR-1 **(NIC)** | 0.38±0.01 | 1.53±0.03 | 4.01±0.07 | 0.90±0.02 | 5.33±0.08 | 5.92±0.02 |
| DMR-4 **(NIC)** | 0.49±0.05 | 2.09±0.14 | 4.25±0.13 | 1.04±0.01 | 6.10±0.40 | 5.87±0.44 |
| DMR-11 **(NIC)** | 0.40±0.01 | 1.73±0.03 | 4.39±0.07 | 0.89±0.01 | 5.60±0.10 | 6.32±0.20 |
| **Example 6** | | | | | | |
| WT | 0.54±0.04 | 1.75±0.10 | 3.28±0.21 | 0.88±0.05 | 3.99±0.42 | 4.51±0.26 |
| DMR-5 **(NF)** | 0.50±0.06 | 2.08±0.23 | 4.16±0.01 | 0.76±0.11 | 5.13±0.23 | 6.97±1.31 |
| DMR-8 **(NF)** | 0.50±0.01 | 1.93±0.08 | 3.90±0.10 | 0.74±0.03 | 5.13±0.08 | 6.92±0.22 |
| DMR-11 **(NF)** | 0.47±0.02 | 2.08±0.03 | 4.46±0.13 | 0.97±0.00 | 5.38±0.03 | 5.57±0.03 |
| **Example 7** | | | | | | |
| WT | 0.54±0.04 | 1.89±0.12 | 4.43±0.43 | 0.93±0.04 | 4.38±0.22 | 4.72±0.17 |
| DMR-17**(NF)** | 0.55±0.03 | 2.40±0.10 | 4.43±0.43 | 0.91±0.06 | 5.23±0.13 | 5.79±0.25 |
| DMR-22**(NF)** | 0.61±0.01 | 2.40±0.15 | 3.93±0.30 | 0.94±0.04 | 5.30±0.15 | 5.63±0.08 |
| **Example 8** | | | | | | |
| WT | 0.56±0.08 | 2.33±0.13 | 4.24±0.35 | 0.86±0.01 | 4.15±0.10 | 4.88±0.06 |
| DMR-37 **(NF)** | 0.68±0.03 | 2.95±0.15 | 4.34±0.03 | 0.96±0.07 | 5.53±0.38 | 5.76±0.03 |
| DMR-39 **(NF)** | 0.62±0.02 | 2.95±0.00 | 4.65±0.03 | 0.92±0.04 | 5.15±0.00 | 5.64±0.22 |

Cells were grown at 60 $\mu$E*m$^{-2}$*s$^{-1}$. All data represent mean values ± SD of at least 3 independent measurements.

Several mutants resistant to norflurazon (NF) or nicotine (NIC) were tested and four species selected on the basis of growth and lutein content.

○ The norflurazon resistant mutant **K-54** (CCAP 211/93) exhibit 41% increased volumetric yield of lutein at the end of deceleration phase;

○ The nicotine resistant mutant **B-7** (CCAP 211/94) increased dry weight lutein content by 45% at the middle of deceleration phase in comparison to the wild strain;

○ The nicotine resistant mutant **DMR-4** (CCAP 211/95) exhibited an increase in the volumetric lutein yield of 36% and in dry weight lutein content of 32% at the middle of deceleration phase and of 62% and 38%, respectively at the end of deceleration phase, as compared to the wild type strain;

○ The best mutant **MR-16** (CCAP 211/96), resistant to nicotine, exhibited an increase in the volumetric lutein yield of 106% (two-fold as compared to the wild type) and in dry weight lutein content of 42% at the end of deceleration phase as compared to the wild type strain.

The four mutants have been deposited at CCAP. The registration numbers are given above in brackets. It is important to underline that the lutein content per dry weight attained values of 5.4 mg g dry weight$^{-1}$ (MR-16) and 5.9 mg g dry weight$^{-1}$ (DMR-4), which are very high. The increase of Lutein production compared to the wild types is presented in Table 2.

**Table 2**

| **Strain** | **Middle declearation phase** | | **End deceleration phase** | |
|---|---|---|---|---|
| | Lutein content | | Lutein content | |
| | Volumetric (mg L$^{-1}$) | Dry weight (mg g$^{-1}$ DW) | Volumetric (mg L$^{-1}$) | Dry weight (mg g$^{-1}$ DW) |
| B7 **(NIC)** | 23 | **45** | 2 | **18** |
| B8 **(NIC)** | 26 | **38** | 17 | 15 |
| K-35 **(NF)** | 17 | 21 | 26 | 30 |
| K-54 **(NF)** | **35** | 23 | **41** | 23 |
| MR-3 **(NF)** | 3 | -11 | **52** | 17 |
| MR-14 **(NIC)** | -7 | -12 | **63** | 29 |
| MR-15 **(NIC)** | 16 | -12 | **45** | 26 |
| MR-16 **(NIC)** | 29 | -11 | **106** | **42** |
| DMR-1 **(NIC)** | -1 | 25 | 42 | 40 |
| DMR-4 **(NIC)** | **36** | 32 | **62** | 38 |
| DMR-11 **(NIC)** | 12 | **37** | 49 | **49** |
| DMR-5 **(NF)** | 19 | 27 | 29 | **55** |
| DMR-8 **(NF)** | 10 | 19 | 29 | **53** |
| DMR-11 **(NF)** | 19 | **36** | 35 | 23 |
| DMR-17 **(NF)** | 27 | 0 | 19 | 23 |
| DMR-22 **(NF)** | 27 | -11 | 21 | 19 |
| DMR-37 **(NF)** | 27 | 2 | 33 | 18 |
| DMR-39 **(NF)** | 27 | 10 | 24 | 16 |

Increase (%) in lutein volumetric and dry weight contents relative to the wild type in liquid cultures.

It is evident that the best mutants we have obtained come from selection based on nicotine resistance. Analysing data of all mutants we have observed a very significant correlation between algal growth rate and lutein volumetric yield or even frequently lutein dry weight content. It is important to remark these mutants exhibited better or similar growth (dry weight) than the wild strain, which is important for practical uses (productivity). This correlation can be used as a criterion for pre-selection of lutein super-producing mutants.

**Claims**

1. A process for obtaining Lutein from algae, **characterised in that**

   (a) green algae selected from a group of mutant strains of *Chlorella sorokiniana* (SAG 211-32) identified by the CCAP deposition references

   (i) CCAP 211-93,
   (ii) CCAP 211-94,
   (iii) CCAP 211-95, and
   (iv) CCAP 211-96

   are cultivated to produce lutein until a desired content of the antioxidant is reached,
   (b) the algae are harvested and used as a product and/or
   (c) the content of lutein is separated off from the remaining biomass.

2. Process according to Claim 1, **characterised in that** mutant strains are used obtained from *Chlorella sorokiniana* after treatment with N-methyl-N'-nitro-nitrosoguanidine (NG).

3. Process according to Claims 1 and/or 2, **characterised in that** cultivation of the algae is conducted at a temperature from 20 to 40 ˚C.

4. Process according to any of the preceding Claims 1 to 3, **characterised in that** cultivation is conducted using a mixotrophic culture medium.

5. Process according to claim 4, **characterised in that** a culture medium is used, comprising 10 to 60 mM nitrates and 20 to 60 mM salts of acetic acid.

6. Process according to any of the preceding Claims 1 to 5, **characterised in that** cultivation is conducted under a light irradiation from 500-1,000 $\mu$E m$^{-2}$ s$^{-1}$.

7. Process according to any of the preceding Claims 1 to 6, **characterized in that** cultivation is conducted under stress.

8. Process according to any of the preceding Claims 1 to 7 **characterised in that** cultivation is conducted in a photo-bioreactor.

9. Process according to any of the preceding Claims 1 to 8 **characterised in that** the cells cultivated in step (b) are collected to form a concentrated suspension, optionally anti-oxidants and emulsifiers are added to said suspension, and the collected cells are optionally disrupted and dried to obtain a lutein or a lutein-enriched product.

10. Use of green algae selected from a group of mutant strains of *Chlorella sorokiniana* (SAG 211-32) identified by the CCAP deposition references

    (i) CCAP 211-93,
    (ii) CCAP 211-94,
    (iii) CCAP 211-95, and
    (iv) CCAP 211-96

    for the production of lutein or lutein-enriched products.

**Patentansprüche**

1. Verfahren für die Gewinnung von Lutein aus Algen, **dadurch gekennzeichnet, dass**

(a) Grünalgen ausgewählt aus einer Gruppe von Stammmutanten von *Chlorella sorokiniana* (SAG 211-32), die gemäß der CCAP-Hinterlegungsreferenzen

(i) CCAP 211-93,
(ii) CCAP 211-94,
(iii) CCAP 211-95 und
(iv) CCAP 211-96

identifiziert sind, kultiviert werden, um Lutein zu produzieren, bis ein gewünschter Gehalt des Antioxidans erreicht ist,
(b) die Algen geerntet und als Produkt verwendet werden und/oder
(c) der Luteingehalt von der verbleibenden Biomasse abgetrennt wird.

2. Verfahren nach Anspruch 1, **dadurch gekennzeichnet, dass** von *Chlorella sorokiniana* nach Behandlung mit N-Methyl-N'-nitronitrosoguanidin (NG) erhaltene Stammmutanten verwendet werden.

3. Verfahren nach den Ansprüchen 1 und/oder 2, **dadurch gekennzeichnet, dass** die Kultivierung der Algen bei einer Temperatur von 20 bis 40˚C erfolgt.

4. Verfahren nach einem der vorhergehenden Ansprüche 1 bis 3, **dadurch gekennzeichnet, dass** die Kultivierung unter Verwendung eines mixotrophen Kulturmediums erfolgt.

5. Verfahren nach Anspruch 4, **dadurch gekennzeichnet, dass** ein Kulturmedium umfassend 10 bis 60 mM Nitrate und 20 bis 60 mM Essigsäuresalze verwendet wird.

6. Verfahren nach einem der vorhergehenden Ansprüche 1 bis 5, **dadurch gekennzeichnet, dass** die Kultivierung unter einer Lichtbestrahlung von 500-1000 $\mu$E m$^{-2}$s$^{-1}$ erfolgt.

7. Verfahren nach einem der vorhergehenden Ansprüche 1 bis 6, **dadurch gekennzeichnet, dass** die Kultivierung unter Stress erfolgt.

8. Verfahren nach einem der vorhergehenden Ansprüche 1 bis 7, **dadurch gekennzeichnet, dass** die Kultivierung in einem Photobioreaktor erfolgt.

9. Verfahren nach einem der vorhergehenden Ansprüche 1 bis 8, **dadurch gekennzeichnet, dass** die in Schritt (b) kultivierten Zellen unter Bildung einer konzentrierten Suspension geerntet werden, die Suspension gewünschtenfalls mit Antioxidantien und Emulgatoren versetzt wird und die geernteten Zellen gewünschtenfalls aufgebrochen und getrocknet werden, wodurch man ein Lutein oder ein mit Lutein angereichertes Produkt erhält.

10. Verwendung von Grünalgen ausgewählt aus einer Gruppe von Stammmutanten von *Chlorella sorokiniana* (SAG 211-32), die gemäß der CCAP-Hinterlegungsreferenzen

(i) CCAP 211-93,
(ii) CCAP 211-94,
(iii) CCAP 211-95 und
(iv) CCAP 211-96

identifiziert sind
für die Produktion von Lutein oder mit Lutein angereicherten Produkten.

**Revendications**

1. Procédé pour obtenir de la lutéine à partir d'algues, **caractérisé en ce que**

(a) on cultive, pour produire de la lutéine jusqu'à obtention d'une teneur souhaitée en antioxydant, des algues vertes choisies dans un groupe de souches mutantes de *Chlorella sorokiniana* (SAG 211-32) identifiées par les numéros de dépôt CCAP suivants :

(i) CCAP 211-93,
(ii) CCAP 211-94,
(iii) CCAP 211-95, et
(iv) CCAP 211-96,

(b) on récolte les algues et on les utilise en tant que produit et/ou
(c) on sépare de la biomasse restante la lutéine présente.

2. Procédé selon la revendication 1, **caractérisé en ce que** les souches mutantes utilisées sont obtenues à partir de *Chlorella sorokiniana* après traitement avec de la N-méthyl-N'-nitro-nitrosoguanidine (NG).

3. Procédé selon la revendication 1 et/ou la revendication 2, **caractérisé en ce que** la culture des algues est réalisée à une température de 20 à 40°C.

4. Procédé selon l'une quelconque des revendications précédentes 1 à 3, **caractérisé en ce que** la culture est réalisée par utilisation d'un milieu de culture mixotrophe.

5. Procédé selon la revendication 4, **caractérisé en ce qu'**on utilise un milieu de culture comprenant des nitrates 10 à 60 mM et des sels de l'acide acétique 20 à 60 mM.

6. Procédé selon l'une quelconque des revendications 1 à 5, **caractérisé en ce que** la culture est réalisée sous exposition à une lumière de 500-1 000 $\mu E\ m^{-2}s^{-1}$.

7. Procédé selon l'une quelconque des revendications précédentes 1 à 6, **caractérisé en ce que** la culture est réalisée en présence d'un stress.

8. Procédé selon l'une quelconque des revendications précédentes 1 à 7, **caractérisé en ce que** la culture est réalisée dans un photobioréacteur.

9. Procédé selon l'une quelconque des revendications 1 à 8, **caractérisé en ce que** les cellules cultivées dans l'étape (b) sont recueillies pour former une suspension concentrée, on ajoute éventuellement à ladite suspension des antioxydants et des émulsifiants, et les cellules recueillies sont éventuellement rompues et séchées pour donner une lutéine ou un produit enrichi en lutéine.

10. Utilisation d'algues vertes choisies dans le groupe de souches mutantes de *Chlorella sorokiniana* (SAG 211-32) identifiées par les numéros de dépôt CCAP

(i) CCAP 211-93,
(ii) CCAP 211-94,
(iii) CCAP 211-95, et
(iv) CCAP 211-96

pour la production de lutéine ou de produits enrichis en lutéine.

**EP 2 157 167 B1**

**Patent documents cited in the description**

- EP 1808483 A

- WO 89006910 A